Europäisches Patentamt

European Patent Office

Office européen des brevets

(19)

(11) Veröffentlichungsnummer : **0 025 611**
**B2**

(12)

# NEUE EUROPÄISCHE PATENTSCHRIFT

(45) Veröffentlichungstag der neuen Patentschrift :
05.03.86

(21) Anmeldenummer : 80200637.9

(22) Anmeldetag : 03.07.80

(51) Int. Cl.⁴ : **A 61 F 13/16**, A 61 F 13/00,
A 41 B 9/12

(54) **Sanitäre Binde.**

(30) Priorität : **30.07.79 DE 2930929**

(43) Veröffentlichungstag der Anmeldung :
**25.03.81 Patentblatt 81/12**

(45) Bekanntmachung des Hinweises auf die Patenterteilung : **17.11.82 Patentblatt 82/46**

(45) Bekanntmachung des Hinweises auf die Entscheidung
über den Einspruch : **05.03.86 Patentblatt 86/10**

(84) Benannte Vertragsstaaten :
**AT BE CH DE FR GB IT LI LU NL SE**

(56) Entgegenhaltungen :
CH-A- 295 532
CH-A- 306 502
DE-A- 1 810 867
DE-A- 2 104 696
DE-A- 2 123 515
DE-U- 6 946 200
FR-A- 2 020 570
FR-A- 2 400 356
GB-A- 781 975
US-A- 3 463 154

(73) Patentinhaber : **UNILEVER NV**
**Burgemeester s'Jacobplein 1 P.O. Box 760**
**NL-3000 DK Rotterdam (NL)**
**BE CH DE FR IT LI LU NL SE AT**
**UNILEVER PLC**
**Unilever House Blackfriars P.O. Box 68**
**London EC4P 4BQ (GB)**
**GB**

(72) Erfinder : **Wallenius, Uwe**
**Marschdamm 2**
**D-2155 York (DE)**
Erfinder : **Schmolling, Dieter**
**Suelldorfer Kirchenweg 1**
**D-2000 Hamburg 55 (DE)**
Erfinder : **Bandzauner, Arnold**
**Olendeelskoppel 16**
**D-2000 Hamburg 65 (DE)**
Erfinder : **Stegmann, Siegfried Hans Fritz**
**Ligusterstieg 37**
**D-2083 Halstenbek (DE)**

(74) Vertreter : **Van Gent, Jan Paulus et al**
**Unilever N.V. Patent Division P.O. Box 137**
**NL-3130 AC Vlaardingen (NL)**

EP 0 025 611 B2

## Beschreibung

Die Erfindung bezieht sich auf ein selbsthaftendes Hygieneprodukt zur Absorption von Körperflüssigkeit, insbesondere auf eine selbsthaftende sanitäre Binde, wie eine Monatsbinde, das auf der Unterseite mit einem druckempfindlichen Kleber zur Erzeugung einer Haftung an einem Kleidungsstück versehen ist. Bequemlichkeitshalber wird in der nachstehenden Beschreibung die Erfindung anhand der besonderen Ausführungsform des selbsthaftenden Hygieneproduktes, der Monatsbinde, näher erörtert ; sie ist aber nicht darauf beschränkt.

Derartige selbsthaftende Monatsbinden sind schon bekannt und haben auch in der Praxis Eingang gefunden.

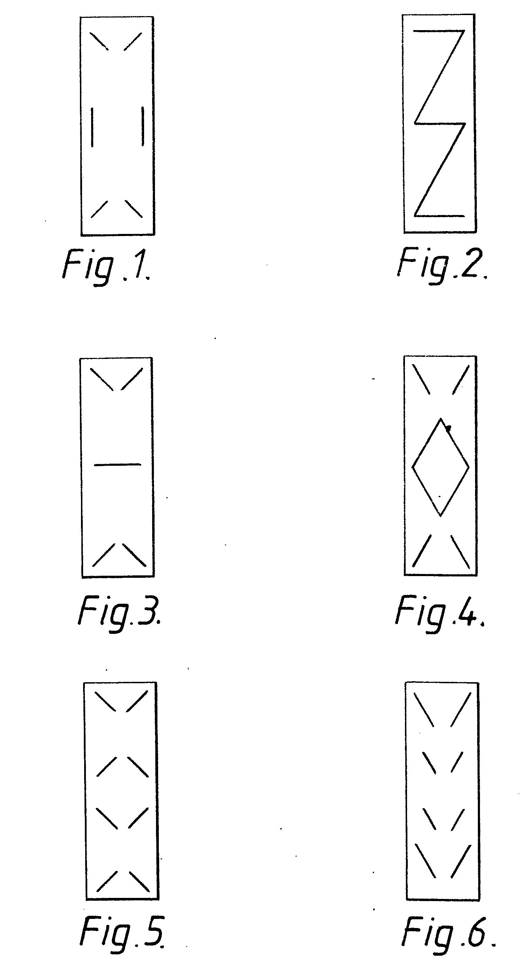

Bei den bekannten Ausführungsformen besteht der Kleber aus einem breiten Streifen, der zentral und in der Längsrichtung auf der Unterseite der Binde angeordnet ist, oder es sind zwei Klebstoffflecken in der Querrichtung in der Nähe der Enden der Binde vorgesehen. Bei letzterer Ausführungsform können auch noch zwei weitere Klebstoffflecken neben den seitlichen Rändern der Binde angeordnet sein. Diese bekannten Ausführungsformen werden in der DE-A-2 123 515, Fig. 4-6, dargestellt. Diese DE-A beschreibt weiter eine selbstklebende Monatsbinde, bei der der Kleber in Form von wenigstens zwei schmalen Linien angeordnet ist, die sich in Längsrichtung auf der Unterseite der Binde erstrecken und zentral zu deren Seiten und Enden verlaufen.

Obwohl verschiedene dieser Monatsbinden in der Praxis einen gewissen Eingang gefunden haben, so haften ihnen noch verbesserungsbedürftige Nachteile an. Bei den meisten Ausführungsformen ist der Kleber nur in einer Dimension entweder in der Längs- oder in der Querrichtung angeordnet worden. Dies kann zwar zu einer ausreichenden Haftung am Kleidungsstück führen, beugt aber dem Problem des Rutschens der Binde bzw. der Quetschfältelung in Längsrichtung nicht in ausreichendem Maße vor. Überdies erfordert eine solche Anordnung einen ziemlich breiten Streifen, der zu einer schwierigen Loslösung der Binde vom Kleidungsstück Anlaß geben kann. Letzteres ist insbesondere der Fall bei derjenigen Ausführungsform, bei der, entlang den Quer- und Längsrändern der Binde umlaufend angeordnet, Klebstoffflecken in der Nähe der Enden und neben den seitlichen Rändern vorgesehen sind, wobei dann die Flecken eine beträchtliche Länge und Breite aufweisen. Dadurch wird oft eine zu große Haftung dieser Binde am Kleidungsstück verursacht, was die Loslösung der Binde, ohne sie zu zerreißen und Klebstoffreste zu hinterlassen, schwierig macht.

Es ist in der FR-A-2 400 356 schon vorgeschlagen worden, das Problem des Rutschens der Monatsbinde zu lösen, indem die Klebstoffstreifen derart auf der Binde angebracht werden, daß die Binde sowohl in der Längsrichtung als auch in der Querrichtung am Kleidungsstück anhaftet. Gemäß diesem Vorschlag sollen die Klebstoffstreifen symmetrisch hinsichtlich eines zentralen Punktes der Längsachse, und in der Nähe dieser Achse auf der Binde angebracht werden, wobei ein Teil der Streifen nicht parallel zu dieser Achse verläuft.

Sämtliche Klebstoffstreifen befinden sich somit gemäß diesem Vorschlag in der Nähe der Längsachse, so daß nur in diesem Gebiet eine Anhaftung der Binde sowohl in Längs- als in Querrichtung am Kleidungsstück erzielt werden kann. Obwohl dadurch das Rutschen der Binde in einem bestimmten Ausmaß verringert werden kann, beugt diese Anordnung der Quetschfältelung der Binde nicht in zufriedenstellender Weise vor.

Es ist daher Aufgabe der Erfindung, gut haftende, jedoch leicht loslösbare Monatsbinden zu schaffen, die sowohl Sitz (Rutschfestigkeit) als auch, und insbesondere, Form (Vermeidung von Quetschfalten) gewährleisten. Diese Aufgabe wird nun ausgehend von dem aus der FR-Al-2 400 356 bekannten selbsthaftenden Hygieneprodukt zur Absorption von Körperflüssigkeit, insbesondere Monatsbinde, das auf der Haftseite mit mehreren schmalen Streifen eines druckempfindlichen Klebers versehen ist, von denen mindestens einige geneigt zur Längsachse des Produkts verlaufen, dadurch gelöst, dass diese Streifen, die von einem Punkt oder mehreren Punkten auf oder in der Nähe der Längsachse ausgehen sich bis in die Nähe der Ecken und/oder der Längsränder des Hygieneprodukts erstrecken.

Durch die Anordnung der Streifen wird eine Drehung der Binde (in der Horizontalebene) um ihre imaginäre X- und Y-Achse beim Tragen erschwert, also die Rutschfestigkeit gewährleistet, und weil sich die Streifen, anders als beim obigen Vorschlag gemäß der FR-A-2 400 356, bis in die Nähe der Längsränder und/oder Ecken der Binde erstrecken, wird dadurch die Quetschfältelung der Binde beim Tragen ebenfalls erschwert. Dem die Tragebequemlichkeit und die Verwendungssicherheit störenden Zusammenfalten der Binde beim Tragen derselben wird somit gemäß der Erfindung vorgebeugt.

Entgegen der ausdrücklichen Vorschrift der obigen FR-A-2 400 356, dass sich die Klebstoffstreifen in der Nähe der Längsachse befinden müssen, sind erfindungsgemäss die geneigt zur Längsachse des Produkts verlaufenden Klebstoffstreifen derart ausgebildet, dass sie sich bis in die Nähe der Ecken und/oder der Längsränder des Hygieneprodukts erstrecken.

Die erfindungsgemäße Anordnung der schmalen Klebstoffstreifen kann auf verschiedene Weisen ausgeführt werden. Die schmalen Streifen erstrecken sich in zumindest zwei verschiedenen Richtungen (wobei sich aber stets mindestens zwei Streifen in der gleichen Richtung erstrecken). Die verschiedenen Richtungen können rechtwinklig zueinander verlaufen, oder sie können unter einem spitzen oder stumpfen Winkel zueinander verlaufen. Letztere Möglichkeit (also nicht rechtwinklig zueinander) wird bevorzugt. Die Klebstoffstreifen brauchen keine geraden Linien zu sein ; sie können auch gekrümmt sein. Wenn sie gekrümmt sind, können sie offen oder geschlossen sein. Kombinationen von geraden und gekrümmten Klebstoffstreifen sind ebenfalls möglich.

Die Streifen können unterbrochen oder ununterbrochen sein ; sie können einander schneiden oder miteinander Vielecke bilden. Im allgemeinen werden die Streifen so angeordnet, daß die Anordnung jeder Hälfte der Binde (entweder in Längs- oder Querrichtung gesehen) ein Spiegelbild der anderen Hälfte ist.

Die Gesamtzahl der Streifen kann weitgehend schwanken ; aus praktischen und ökonomischen Gründen aber wird die Anzahl der Streifen so klein wie möglich gehalten.

Die Dimensionen der Streifen sind von der erwünschten Anordnung abhängig ; ihre Breite beträgt im allgemeinen von 0,2 bis 20, insbesondere 0,5 bis 8, und vorzugsweise 0,8 bis 5 mm. Die Breite der Streifen ist zu einem gewissen Grad von der Oberfläche des Hygieneproduktes abhängig ; so können bei z. B. größeren Einlagen die Streifen eine Breite im Obergebiet des angegebenen Breitebereiches von 0,2 bis 20 mm haben, während dagegen bei z. B. Monatsbinden die Streifen vorzugsweise eine Breite haben, die im Untergebiet des angegebenen allgemeinen Bereiches liegt. Ihre Länge wird im allgemeinen 0,5 cm bis 20 cm betragen ; dies wird vor allem durch die Anordnung, die Dimensionen der Binde und die Art des Streifens bedingt. Die Streifen erstrecken sich von einem Punkt oder von mehreren Punkten, auf oder in der Nähe der Längsachse, bis in die Nähe der Längsränder und/oder Ecken der Binde, und hierdurch, als auch durch die Dimensionen läßt sich die erwünschte Länge der Streifen leicht bestimmen.

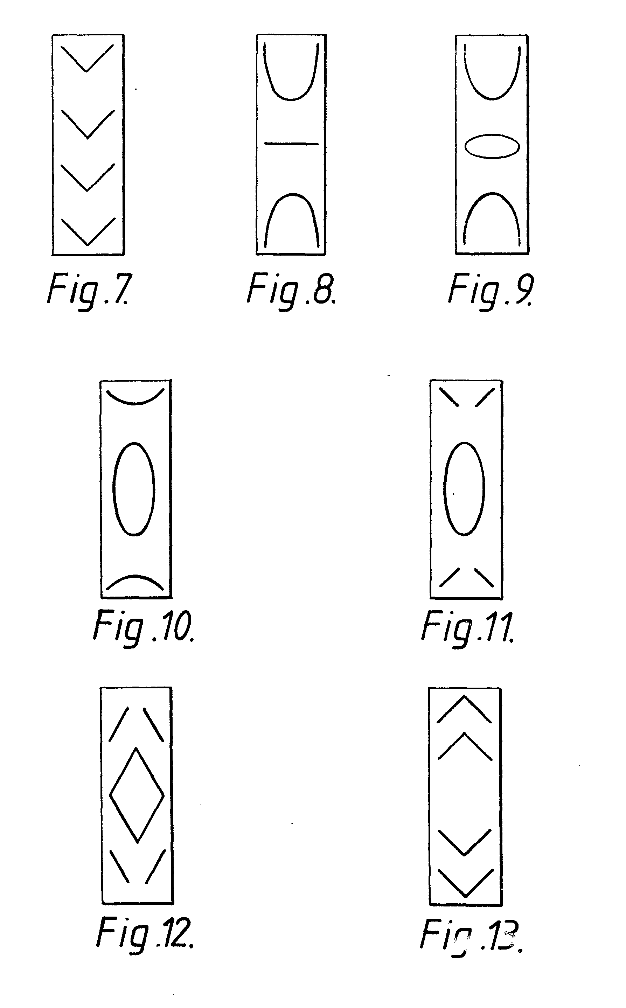

Typische Beispiele von Anordnungen der Klebstoffstreifen gemäß der Erfindung werden durch die Zeichnungen näher erörtert. Die Zeichnungen (Fig. 1 bis 13) stellen Unteransichten von Monatsbinden dar, mit der Anordnung der schmalen Klebstoffstreifen ohne Schutzfolie gezeichnet. Besonders bevorzugte Anordnungen sind diejenigen gemäß den Fig. 2, 4, 5 und 7.

Die Erfindung ist auf selbsthaftende Monatsbinden und andere Körperflüssigkeit/Sekrete absorbierende Produkte und Einlagen anwendbar. Im allgemeinen bestehen die Monatsbinden grundsätzlich aus einem länglichen, dünnen Kissen aus absorptionsfähigem Material, das entweder völlig oder teilweise von einer Hülle aus flüssigkeitsdurchlässigem Material umschlossen ist.

Die Klebstoffstreifen werden mittels üblicher Auftragverfahren auf der Haftseite des Kissens angebracht, entweder durch direktes Anbringen des Klebstoffes in der gewünschten Anordnung oder durch Aufbringen einer oder mehrerer Schutzfolien, die zuvor an ihrer Auftragseite mit noch feuchtem Kleber versehen worden ist bzw. sind, wodurch die Klebstoffstreifen zugleich mit den Schutzfolien angebracht werden. Die Klebstoffstreifen werden üblicherweise mit einer oder mehreren Schutzfolien abgedeckt.

Der Kleber soll derart ausgewählt werden, daß eine ausreichende Haftung am Kleidungsstück erreicht wird, die Binde jedoch ohne Zerreißen losgelöst werden kann.

Durch die erfindungsgemäße Anordnung der schmalen Klebstoffstreifen wird dem Rutschen der Monatsbinde weitgehend vorgebeugt und eine leicht loslösbare Monatsbinde erhalten. Auch ein mögliches Einengen oder Wölben der Monatsbinde (Quetschfaltung) wird durch die erfindungsgemäße Anordnung signifikant verringert.

**Patentansprüche**

1. Selbsthaftendes Hygieneprodukt zur Absorption von Körperflüssigkeit, insbesondere Monatsbinde, das auf der Haftseite mit mehreren schmalen Streifen eines druckempfindlichen Klebers versehen ist, von denen mindestens einige geneigt zur Längsachse des Produkts verlaufen, dadurch gekennzeichnet, dass diese Streifen, die von einem Punkt oder mehreren Punkten auf oder in der Nähe der Längsachse ausgehen, sich bis in die Nähe der Ecken und/oder der Längsränder des Hygieneprodukts erstrecken.

2. Hygieneprodukt nach Anspruch 1, dadurch gekennzeichnet, dass die schmalen Streifen sich zumindest in zwei verschiedenen Richtungen erstrecken.

3. Hygieneprodukt nach Anspruch 2, dadurch gekennzeichnet, dass die schmalen Streifen sich in Richtungen rechtwinklig zueinander erstrecken.

4. Hygieneprodukt nach Anspruch 2, dadurch gekennzeichnet, dass die schmalen Streifen sich in Richtungen, die einen spitzen oder stumpfen Winkel miteinander bilden, erstrecken.

5. Hygieneprodukt nach den Ansprüchen 1 bis 4, dadurch gekennzeichnet, dass die Streifen entweder gerade oder gekrümmt (offen oder geschlossen) sind.

6. Hygieneprodukt nach den Ansprüchen 1 bis 5, dadurch gekennzeichnet, dass die geneigt verlaufenden Streifen, bezogen auf die Längs- oder Querachse der Haftseite, spiegelbildlich angeordnet sind.

**Claims**

1. A self-adhering sanitary product for the absorption of body fluids, in particular sanitary towels, provided on the adhesive side with several narrow strips of a pressure-sensitive adhesive, at least some of which run inclined towards the longitudinal axis of the product, characterized in that these strips, which start from one or more points on or near the longitudinal axis, extend into the vicinity of the corners and/or the longitudinal edges of the sanitary product.

2. A sanitary product according to Claim 1, characterized in that the narrow strips extend in at least two different directions.

3. A sanitary product according to Claim 2, characterized in that the narrow strips extend in directions at right angles to each other.

4. A sanitary product according to Claim 2, characterized in that the narrow strips extend in directions forming an acute or obtuse angle with each other.

5. A sanitary product according to Claims 1 to 4, characterized in that the strips are either straight or curved (open or closed).

6. A sanitary product according to Claims 1 to 5, characterized in that the strips running inclined with respect to the longitudinal or transverse axis of the adhesive side, are arranged in mirror image.

**Revendications**

1. Produit hygiénique autoadhésif destiné à absorber des humeurs corporelles, en particulier serviette périodique, dont la face adhésive est pourvue de plusieurs bandes étroites d'un adhésif sensible à la pression parmi lesquelles quelques-unes au moins sont inclinées par rapport à l'axe longitudinal du produit, caractérisé en ce que ces bandes, qui partent d'un point ou de plusieurs points situés sur l'axe longitudinal ou au voisinage de celui-ci s'étendent jusqu'à proximité des coins et/ou des bords longitudinaux du produit hygiénique.

2. Produit hygiénique suivant la revendication 1, caractérisé en ce que les bandes étroites s'étendent dans au moins deux directions différentes.

3. Produit hygiénique suivant la revendication 2, caractérisé en ce que les bandes étroites s'étendent dans des directions perpendiculaires entre elles.

4. Produit hygiénique suivant la revendication 2, caractérisé en ce que les bandes étroites s'étendent dans des directions formant entre elles un angle aigu ou un angle obtus.

5. Produit hygiénique suivant l'une quelconque des revendications 1 à 4, caractérisé en ce que les bandes sont droites ou incurvées (ouvertes ou fermées).

6. Produit hygiénique suivant l'une quelconque des revendications 1 à 5, caractérisé en ce que les bandes qui sont inclinées sont disposées de manière énantiomorphe par rapport à l'axe longitudinal ou à l'axe transversal du côté adhésif.

Fig .1.

Fig.2.

Fig.3.

Fig.4.

Fig.5

Fig.6.

Fig.7.

Fig.8.

Fig.9.

Fig.10.

Fig.11.

Fig.12.

Fig.13.